Europäisches Patentamt

European Patent Office

Office européen des brevets

(11) Publication number: **0 155 779**
**B1**

(12) **EUROPEAN PATENT SPECIFICATION**

(45) Date of publication of patent specification: **26.04.89**

(51) Int. Cl.⁴: **C 07 C 103/19, C 07 C 102/00**

(21) Application number: **85301285.4**

(22) Date of filing: **26.02.85**

(54) **A method for the optical purification of an optically active 2,2-dimethylcyclopropanecarboxylic acid amide.**

(30) Priority: **28.02.84 JP 37491/84**
**21.03.84 JP 54633/84**

(43) Date of publication of application:
**25.09.85 Bulletin 85/39**

(45) Publication of the grant of the patent:
**26.04.89 Bulletin 89/17**

(84) Designated Contracting States:
**CH DE FR GB IT LI**

(56) References cited:
**EP-A-0 028 778**
**GB-A-1 260 847**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Minai, Masayoshi**
**1606-5, Harimatamachi**
**Moriyama-Shi (JP)**
Inventor: **Katsura, Tadashi**
**9-20-911, Yamadahigashi-2-chome**
**Suita-Shi (JP)**
Inventor: **Ueda, Yuji**
**171-26, Ikegamicho**
**Izumi-Shi (JP)**

(74) Representative: **Harrison, David Christopher et al**
**MEWBURN ELLIS & CO 2/3 Cursitor Street**
**London EC4A 1BQ (GB)**

Courier Press, Leamington Spa, England.

# EP 0 155 779 B1

**Description**

The present invention relates to a method for obtaining an optically active 2,2 - dimethylcyclopropanecarboxamide having optically high purity from optically active mixtures of 2,2 - dimethylcyclopropanecarboxamide in which either one of the optically active isomers is present in excess.

An optically active 2,2 - dimethylcyclopropanecarboxamide (hereinafter referred simply to as carboxamide) is a very useful compound, for example, as an intermediate for dehydropeptidase I inhibitor, and a high optical purity is required also for this purpose.

This carboxamide can be converted to an optically active 2,2 - dimethylcyclopropylamine with sodium hypochlorite, and this amine is a useful compound as an optical resolving agent or an amine for measurement optical purity.

The conventionally well-known method for obtaining such optically active carboxamide is a method in which for example dl - 2,2 - dimethylcyclopropanecarboxylic acid is optically resolved into optically active 2,2 - dimethylcyclopropanecarboxylic acid, and the resulting acid is converted to acid chloride and then amidated [Japanese Patent Application Kokai (Laid-open) No. 81518/1981]. This method uses an optically active 2,2 - dimethylcyclopropanecarboxylic acid as a material, so that there is a problem that the compound should be made of optically high purity.

Also, the optically active mixture of the carboxamide in which either one of the optically active isomers is present in excess, can be obtained, for example, by the method described in U.S. Patent No. 4,029,690, that is the amidation of 2,2 - dimethylcyclopropanecarboxylic acid containing either one of the optically active isomers in excess. But, nothing is known about a method to obtain a high-purity optically active acid amide from such optically active mixture.

But, in order to obtain the optically active carboxamide, if there is a method in which the excess optically active carboxamide only can easily be separated from such the optically active mixture, its industrial value becomes very high.

The present inventors, in the course of an extensive study to obtain the optically active carboxamide, found that the dl-carboxamide has a higher melting point and a lower solubility in solvents than those of the optically active carboxamide (for example, the solubility in methyl isobutyl ketone at 25°C is 0.5 to 0.8% for the dl-form and 3.2 to 3.5% for the d-isomer). This result means that, when the optically active mixture of the carboxamide is recrystallized, the carboxamide obtained as crystal will be poor in optical purity, and besides that it will be difficult to obtain the optically active carboxamide of high optical purity from the filtrate. .

Generally, in the optical purification by recrystallization of an optically active mixture in which either one of the optically active isomers is present in optical excess, a high-purity optically active isomer is obtained at the crystal side, there being few examples in which said high-purity optically active isomer is obtained at the filtrate side. In fact, the recrystallization of optically active mixtures of the carboxamide by the normal method also gives no optically active carboxamide of high optical purity at any of the crystal and filtrate sides.

For this reason, the present inventors extensively studied to separate the optically active carboxamide with high purity and good yields from optically active mixtures of the carboxamide in which either one of the optically active isomers is present in optical excess. As a result, the present inventors found that: The dl-carboxamide is dissolved in only an extremely small amount in a solution saturated with the optically active carboxamide or containing the carboxamide in concentrations near to saturation; and the dl-carboxamide is not only not dissolved in such solution, but also deposited as crystal from its solution when the optically active carboxamide is dissolved in the solution in amounts equal or near to saturation. As a result of a further study based on this information, the present inventors attained to the present invention.

An object of the present invention is to provide a method for obtaining an optically active 2,2 - dimethylcyclopropanecarboxamide by dissolving an optically active mixture of 2,2 - dimethylcyclopropanecarboxamide in which either one of the optically active isomers is present in excess, in a solvent with heating, cooling the solution to deposit the racemate, filtering off the racemate and then recovering said optically active isomer from the filtrate, which method is characterized in that the amount of the solvent used is in the range of from 0.75 to 1.3 times as much as that required for the excess optically active isomer present in said optically active mixture to form a saturated solution at a temperature at which the racemate crystal is filtered off.

The present invention will be illustrated hereinafter in detail.

The optically active mixture of 2,2 - dimethylcyclopropanecarboxamide in which either one of the optically active isomers is present in excess, as used as a starting material in the present invention, is obtained, for example, by amidation of an optically active mixture of 2,2 - dimethylcyclopropanecarboxylic acid containing either one of the optically active isomers in excess. For this purpose, a method is known in which said optically active mixture of 2,2 - dimethylcyclopropanecarboxylic acid is reacted with oxalyl chloride in an organic solvent, and after removing the solvent by evaporation, the resulting acid chloride is dissolved in methylene chloride, and then ammonia gas is bubbled into the solution to attain the amidation [Japanese Patent Application Kokai (Laid-open) No. 81518/1981].

But, this method is not satisfactory as an industrial method, because oxalyl chloride used as a material

2

is expensive and ammonium chloride sparingly soluble in the oganic solvent is produced as by-product so that separation of the intended compound from the reaction mixture is difficult, and besides because the yield is not always high.

For this reason, such a method is preferred that cheap thionyl chloride is usable in acid-chlorination of the carboxylic acid, and that ammonia, particularly aqueous ammonia, is usable in acid-amidation of the resulting acid chloride.

Said optically active mixture of 2,2 - dimethylcyclopropanecarboxylic acid in which either one of the optically active isomers is present in excess, as used as a starting material in this reaction, can be produced, for example, by the method described in the specification of U.S. Patent No. 4,029,690.

In the reaction of 2,2 - dimethylcyclopropanecarboxylic acid with thionyl chloride, a solvent is not particularly necessary, but may be used as need arises.

In the case where a solvent is used, the kind of the solvent is not particularly limited, so far as it gives no adverse effect on the reaction. As the solvent, there are given for example, ethers, ketones, aliphatic or aromatic hydrocarbons, halogenated hydrocarbons such as ethyl ether, tetrahydrofuran, n-hexane, cyclohexane, benzene, chloroform, dichloroethane, dichloromethane, acetone and the like. These solvents may be used in a mixture of two or more of them.

The amount of the solvent is not also particularly limited, but generally, it is 0.5 to 10 times by weight based on 2,2 - dimethylcyclopropanecarboxylic acid which is a material.

Considering the subsequent amidation, however, there is no special need to use an organic solvent, and it is most preferred to react 2,2 - dimethylcyclopropanecarboxylic acid with thionyl chloride without a solvent and advance toward the subsequent amidation using the reaction mixture as obtained.

For this reason, it is also preferred that the molar ratio of 2,2 - dimethylcyclopropanecarboxylic acid to thionyl chloride is in the range of 1 to 1.02—1.3. When amounts of thionyl chloride in excess of this range were used, therefore, it becomes necessary to recover or remove the excess thionyl chloride from the reaction solution for reasons of economy and countermeasure for environmental pollution.

The reaction temperautre is in the range of from 0° to 150°C, preferably from 10° to 100°C.

By such reaction, 2,2 - dimethylcyclopropanecarboxylic acid chloride is easily obtained in high yields.

2,2 - Dimethylcyclopropanecarboxylic acid chloride thus obtained is amidated by reaction with ammonia, and it is preferred to use aqueous ammonia as ammonia and carry out the amidation in the presence or absence of an organic solvent sparingly soluble in the aqueous ammonia.

The concentration of aqueous ammonia used herein is in the range of, generally, from 5 to 25% and preferably from 8 to 20%, and the amount of aqueous ammonia is from 1.5 to 6 times by mole and preferably from 1.8 to 4 times by mole, as converted to ammonia, based on 2,2 - dimethylcyclopropanecarboxylic acid chloride.

2,2 - Dimethylcyclopropanecarboxamide produced by this reaction is a water-soluble substance, and therefore, in the practice of this amidation, it is preferred to use a solvent which can dissolve 2,2 - dimethylcyclopropanecarboxamide with a high solubility and besides can be applied to both extraction and recovery of said carboxamide from the reaction solution after period of the amidation. Also, said solvent is further more preferred if it can be used in the final step wherein the optically active carboxamide is separated from the 2,2 - dimethylcyclopropanecarboxamide obtained above containing said optically active carboxamide in optical excess.

Preferred solvents usable for this purpose include ones sparingly soluble in water such as ketones (e.g. methyl ethyl ketone, methyl isobutyl ketone, cyclopentanone, cyclohexanone), halogenated hydrocarbons (e.g. dichloromethane, chloroform, dichloroethane, tetrachloroethane, dichlorobenzene), esters (e.g. ethyl acetate, isopropyl acetate, methyl propionate, pentyl acetate) and the like.

The reaction temperature is preferably in the range of from −10° to 30°C, more preferably from −10° to 15°C.

It may be possible to carry out the amidation without a solvent and after period of the reaction, carry out extraction with a solvent to obtain the objective compound. But, coexistence of a solvent in the system is preferred in terms of the yield.

After period of the reaction, the recovery of 2,2 - dimethylcyclopropanecarboxamide from the reaction mixture may be carried out by neutralizing the mixture with hydrochloric acid or sulfuric acid if necessary and heating the mixture to dissolve and extract the carboxamide in the organic solvent. The aqueous layer is re-extracted with a solvent if necessary; the organic layers are combined and concentrated; an inert solvent such as heptane, hexane or the like is added to the concentrated solution to deposit crystals or the concentrated solution is cooled to deposit crystals; and then the crystal is filtered off. By this procedure, the optically active mixture of 2,2 - dimethylcyclopropanecarboxamide containing either one of the optically active isomers in optical excess can be obtained in good yields.

For the subsequent optical purification for obtaining the objective optically active 2,2 - dimethylcyclopropanecarboxamide from said optically active mixture of 2,2 - dimethylcyclopropanecarboxamide obtained above, the organic solvent solution of said optically active mixture obtained above may be used as such.

Explanation will be given to said optical purification. The method of the present invention is a one including dissolving an optically active mixture of 2,2 - dimethylcyclopropanecarboxamide in which either one of the optically active isomers is present in excess, in a solvent with heating, cooling the solution to deposit the racemate, filtering off the racemate and then recovering the optically active isomer from the filtrate, in which method the optically active 2,2 - dimethylcyclopropanecarboxamide is obtained in an extremely high optical purity by using the solvent in amounts ranging from 0.75 to 1.3 times as much as that required for said excess optically active isomer present in said optically active mixture to form a saturated solution at a temperature at which the racemate crystal is filtered off.

Any solvent may be used without limitation, if it is inert to the carboxamide and has an ability to dissolve even a small amount of the acid amide. But, more preferred solvents in terms of efficiency are such that the solubility of the optically active carboxamide in them is as high as possible and that of the dl-carboxamide in them is as low as possible. The most preferred solvent is such that a difference in solubility between the optically active acid amide and dl-carboxamide is large and besides the solubility of the optically active carboxamide is relatively large.

As such solvent, there may be given for example alcohols (e.g. methanol, ethanol, isopropyl alcohol, butanol), ketones (e.g. acetone, methyl ethyl ketone, methyl isobutyl ketone, cyclopentanone, cyclohexanone), esters (e.g. ethyl acetate, isopropyl acetate, methyl propionate, pentyl acetate), halogenated hydrocarbons (e.g. dichloromethane, chloroform, dichloroethane, tetrachloroethane, dichlorobenzene), ethers (e.g. tetrahydrofuran, dioxane) and the like. These solvents may contain water.

The amount of such solvent used is very important to the present invention, and it is 0.75 to 1.3 times, preferably 0.75 to 1.2 times as much as that required for the excess optically active isomer to form a saturated solution at a temperature at which the racemate crystal is filtered off.

But, even if the amount of solvent is in the foregoing range, too small or too large amounts of the solvent are not preferred in terms of operation and productivity. Generally, the kind of solvent and temperature are properly selected, and under that condition, the amount of the solvent is determined so that the foregoing range is satisfied and besides said amount is 3 to 100 times by weight based on the carboxamide which is a material.

The operation temperatures for dissolution to crystal filtration are optionally selected within the range of from freezing point to boiling point of the solvent used. Generally, however, the lower limit of the temperature is −20°C. By using an autoclave, even temperatures higher than the boiling point of the solvent at atmospheric pressure may be applied, but not practically so advantageous.

In the practice of the present invention, the amount of the solvent for dissolving the optically active mixture and the temperature at which the carboxamide racemate deposited by cooling the resulting solution is filtered off, should be properly determined.

Generally, the amount of the solvent is made rather small when the acid amide racemate is filtered off at a relatively high temperature, while it is made rather large when the filtration temperature is low.

The normal operation for the practice of the present invention will be explained hereinafter.

An optically active mixture of the carboxamide containing either one of the optically active isomers in optical excess is dissolved in a proper amount of a solvent by heating. In this case, it is not always necessary to dissolve said mixture completely at this heating temperature, so that a part of the acid amide racemate may then remain undissolved as crystals. Thereafter, the solution is slowly cooled, causing that carboxamide racemate which is dissolved to be deposited. The deposited carboxamide racemate and any previously undissolved racemate is filtered off at a proper temperature.

Recovery of the optically active carboxamide from the filtrate is carried out, for example, by concentrating the filtrate, adding a solvent not dissolving the optically active carboxamide (e.g. hexane, heptane, petroleum ether, ligroin) to the concentrated solution or cooling said solution to deposit the crystal of the optically active carboxamide and then collecting the crystal by filtration.

In this step, when the carboxamide-containing organic layer obtained in the preceding amidation is used, the organic layer is first properly concentrated to adjust the amount of the organic solvent to 0.75 to 1.3 times as much as that necessary to form the essentially saturated solution of the excess isomer at the filtration temperature. Thereafter, the organic layer is heated to turn it into solution and then subjected to crystallization (crystallization of the carboxamide racemate) in the same manner as above.

Thus, the optically active carboxamide of extremely high optical purity can be obtained by the method of the present invention.

The solvent used in the present invention is as described above, but it is advantageous industrially to use the same solvent as used in producing the optically active mixture of the carboxamide which is a starting material of the present invention.

The present invention will be explained hereinafter with reference to the following examples.

Reference Example 1

To a four-necked flask equipped with a thermometer, a stirrer and a dropping funnel were added 223.16 g of 14% aqueous ammonia and 400 g of methyl isobutyl ketone, and 80 g of 2,2 - dimethylcyclopropanecarboxylic acid chloride having an optical purity of 82.5% was added dropwise at −5° to 5°C over 3 hours. After period of the dropwise addition, the reaction solution was kept at the same temperature for 1 hour.

The reaction solution, after neutralized to a pH of 7 with conc. sulfuric acid, was heated to 80°C and separated into an oily and aqueous layers at the same temperature. Thereafter, 200 g of methyl isobutyl ketone was added to the aqueous layer to carry out extraction, and the system was separated into an oily

4

and aqueous layers. The oily layers obtained (methyl isobutyl ketone layer) were combined and washed with water, and after concentration, hexane was added to the layer. The deposited crystal was collected by filtration and dried to obtain 67.6 g of 2,2 - dimethylcyclopropanecarboxamide having an optical purity of 82.5%.

Example 1

To a four-necked flask equipped with a thermometer, a stirrer, a condenser and a dropping funnel was added 114.1 g (1 mole) of 2,2 - dimethylcyclopropanecarboxylic acid having an optical purity of 82.5%, 121.3 g (1.02 mole) of thionyl chloride was added dropwise at 60° to 70°C over 4 hours, and the reaction solution was kept at the same temperature for 2 hours. After period of the reaction, the reaction solution was cooled and analyzed to find that 135.9 g of 2,2 - dimethylcyclopropanecarboxylic acid chloride (purity, 96.2%; yield, 98.6%) was obtained in the solution.

Thereafter, 20.8 g of this reaction solution [corresponding to 20 g (0.15 mole) of 2,2 - dimethylcyclopropanecarboxylic acid chloride] was added dropwise to a mixed solution of 50.3 g (0.47 mole) of 16% aqueous ammonia and 80 g of cyclohexanone at −5° to 3°C over 6 hours, and then the reaction solution was kept at the same temperature for 1 hour. After period of the reaction, the reaction solution was neutralized to a pH of 6.5 with conc. sulfuric acid, and after heated to 75°C, separated into an oily and aqueous layer. The aqueous layer was extracted with 50 g of cyclohexanone, and the oily layer separated was combined with the preceding oily layer. The mixed oily layer was washed with water, cooled to 35°C and aged at the same temperature, and the deposited crystal was collected by filtration at the same temperature.

The crystal obtained by filtration was a crude cake containing 2,2 - dimethylcyclopropanecarboxamide having an optical purity of 28.5%.

The filtrate was concentrated under reduced pressure, and heptane was added to the residual solution. The deposited crystal was collected by filtration and dried under reduced pressure to obtain 12.8 g of (+) - 2,2 - dimethylcyclopropanecarboxamide having an optical purity of 98.5%.

m.p. 135.5°—136.5°C

$[\alpha]_{546}^{20}$ +99.3° (c=2, ethanol).

Example 2

To a four-necked flask equipped with a thermometer, a stirrer and a condenser were added 5 g of 2,2 - dimethylcyclopropanecarboxamide having an optical purity of 82.5% obtained in Reference Example 1 and 176 g of methyl isobutyl ketone (MIBK), and the resulting mixture was heated to turn it into a solution and then cooled to 10°C. The deposited crystal was filtered off at the same temperature to obtain 2,2 - dimethylcyclopropanecarboxylic acid amide having an optical purity of 20.1%. The filtrate was concentrated, hexane was added to the residual solution, and the deposited crystal was collected by filtration and dried to obtain 4.03 g (percent recovery, 80.6%) of (+) - 2,2 - dimethylcyclopropanecarboxamide having an optical purity of 97.5%.

m.p. 137°—139°C

$[\alpha]_{546}^{20}$ +98° (c=2, ethanol).

Hereupon, the amount of MIBK used in this example is 1.25 times as much as that required for excess (+) - 2,2 - dimethylcyclopropanecarboxamide present in the solution to from a saturated solution at the temperature (10°C) at which the crystal is filtered off.

In the examples described later, all the amounts of MIBK simply expressed by a multiple have the same meaning as above.

Examples 3 to 5 and Comparative Examples 1 and 2

Procedure was carried out in the same manner as in Example 2 except that the amounts of MIBK and crystal filtering temperatures shown in Table 1 were used. The results are shown in Table 1.

TABLE 1

| | | Amount of MIBK | Filtration temperature (°C) | Crystal side | Filtrate side [(+)-isomer crystal] | |
|---|---|---|---|---|---|---|
| | | | | Optical purity (%) | Optical purity (%) | Percent recovery (%) |
| Example | 3 | 140 g (1.0 time) | 10 | 23.7 | 98.5 | 78.6 |
| | 4 | 117 g (0.83 time) | 10 | 33 | 99 | 75 |
| | 5 | 65 g (0.83 time) | 30 | 30 | 99 | 76 |
| Comparative Example | 1 | 230 g (1.67 time) | 10 | 15 | 90 | 90 |
| | 2 | 70 g (0.5 time) | 10 | 62 | 100 | 54 |

Example 6

To a four-necked flask equipped with a thermometer, a stirrer and a condenser were added 5 g of 2,2 - dimethylcyclopropanecarboxamide having an optical purity of 82.5% obtained in Reference Example 1 and 100 g (0.83 time) of aqueous methyl isobutyl ketone (water content, 2.5—3%) showing saturation with water at 20°C, and the resulting mixture was heated to turn it into a solution and then cooled to 20°C. The deposited crystal was filtered off at the same temperature to obtain 2,2 - dimethylcyclopropanecarboxamide having an optical purity of 16.5%. The filtrate was concentrated, hexane was added to the residual solution, and the deposited crystal was collected by filtration and dried to obtain 4 g (percent recovery, 80%) of (+) - 2,2 - dimethylcyclopropanecarboxamide having an optical purity of 99%.

m.p. 137.5°—139°C

$[\alpha]_{546}^{20}$ +99° (c=2, ethanol).

Example 7

To a four-necked flask equipped with a thermometer, a stirrer, a condenser and a dropping funnel was added 114.1 g (1 mole) of 2,2 - dimethylcyclopropanecarboxylic acid (optical purity, 80%), 121.3 g (1.02 mole) of thionyl chloride was added dropwise at 60° to 70°C over 2 hours, and the reaction solution was kept at the same temperature for 4 hours.

This reaction solution was added dropwise from the dropping funnel at −2° to 5°C over 2 hours to a mixed solution of 454.4 g (3.74 moles) of 14% aqueous ammonia and 500 g of MIBK fed to the same flask as above. The reaction solution was kept at the same temperature for 30 minutes and neutralized to a pH of 7 or less with sulfuric acid. Thereafter, the solution was then heated to 75° to 80°C to turn it into solution and separated into two layers. The separated aqueous layer was re-extracted with 300 g of MIBK.

The MIBK layers were combined, washed with water, cooled to 30°C and aged at the same temperature. The deposited crystal was collected by filtration at the same temperature.

The crystal obtained by filtration was a crude cake containing 2,2 - dimethylcyclopropanecarboxamide having an optical purity of 19.8%.

The filtrate was concentrated under reduced pressure, hexane was added to the residual solution and the deposited crystal was collected by filtration and dried under reduced pressure to obtain 87.24 g of (+) - 2,2 - dimethylcyclopropanecarboxamide having an optical purity of 98%.

m.p. 136°—137.5°C

$[\alpha]_{546}^{20}$ +99° (c=2, ethanol).

Example 8

A mixture of 20 g of MIBK and 2 g of 2,2 - dimethylcyclopropanecarboxamide having an optical purity of 31.5%, as prepared by mixing 1 g each of the filtered crystals obtained in Examples 3 and 4, was stirred at 70°C for 1 hour to dissolve the crystal. The resulting solution was slowly cooled to 25°C and stirred at the same temperature for 30 minutes. The deposited crystal was collected by filtration and dried under reduced pressure to obtain 2,2 - dimethylcyclopropanecarboxamide having an optical purity of 6.7%.

MIBK was removed from the filtrate by evaporation, and the residue was dried to obtain 0.57 g of (+) - 2,2 - dimethylcyclopropanecarboxamide having an optical purity of 97%.

$[\alpha]_{546}^{20}$ +97° (c=2, ethanol).

Example 9

Procedure was carried out in completely the same manner as in Example 7 except that 2,2 - dimethylcyclopropanecarboxylic acid having an optical purity of 90% was used in place of 2,2 - dimethylcyclopropanecarboxylic acid having an optical purity of 80%, and besides that the amounts of MIBK at the respective scenes of use were 565 g and 282.5 g, to obtain 2,2 - dimethylcyclopropanecarboxamide having an optical purity of 46% as a filtered crystal. From the filtrate was obtained 93.9 g of (+) - 2,2 - dimethylcyclopropanecarboxamide having an optical purity of 99% as a crystal.

m.p. 136°—137.5°C

$[\alpha]_{546}^{20}$ +99.5° (c=2, ethanol).

Example 10

A mixture of 100 g of ethyl acetate and 5 g of 2,2 - dimethylcyclopropanecarboxamide having an optical purity of 82.5% obtained in Reference Example 1, was kept under reflux for 1 hour and then slowly cooled to 30°C. The deposited crystal was filtered off at the same temperature. The filtrate was concentrated as such, and hexane was added to the residual solution. The deposited crystal was collected by filtration and dried to obtain 3.65 g of (+) - 2,2 - dimethylcyclopropanecarboxamide having an optical purity of 98.3%.

$[\alpha]_{546}^{20}$ +99.2° (c=2, ethanol).

Example 11

Procedure was carried out in completely the same manner as in Example 10 except that 250 g of chloroform was used in place of ethyl acetate, to obtain 3.4 g of (+) - 2,2 - dimethylcyclopropanecarboxamide having an optical purity of 97.9%.

Example 12

A mixture of 50 g of 20% ethanol and 5 g of 2,2 - dimethylcyclopropanecarboxamide having an optical purity of 82.5% obtained in Reference Example 1, was kept at 80°C for 1 hour and then slowly cooled to 30°C. After maintaining the same temperature for 1 hour, the deposited crystal was filtered off. The filtrate was concentrated as such, and additional ethanol was then added to the residual solution to carry out azeotropic dehydration. Thereafter, hexane was added to the residual solution, and the deposited crystal was collected by filtration and dried to obtain 3.80 g of (+) - 2,2 - dimethylcyclopropanecarboxamide having an optical purity of 97.3%.

$[\alpha]_{546}^{20}$ +97.7° (c=2, ethanol).

Example 13

To the same apparatus as used in Example 1 were added 50.3 g of 16% aqueous ammonia and 90 g of pentyl acetate, and 20.8 g of 2,2 - dimethylcyclopropanecarboxylic acid chloride obtained in Example 1 was added dropwise at −5° to 0°C over 3 hours. Thereafter, the reaction solution was kept at the same temperature for 1 hour. After period of the reaction, the reaction solution was neutralized to a pH of 6.5 with conc. sulfuric acid, and after heating to 70° to 75°C, separated into an oily and aqueous layers. After extracting the aqueous layer with 70 ml of pentyl acetate, the oily layer separated and the preceding oily layer were combined, washed with water, cooled to 35°C and aged at the same temperature. The deposited crystal was collected by filtration at the same temperature to obtain a crude cake containing 2,2 - dimethylcyclopropanecarboxamide having an optical purity of 41.5%.

The filtrate was concentrated under reduced pressure, and petroleum ether was added to the residual solution. The deposited crystal was collected by filtration and dried under reduced pressure to obtain 11.9 g of (+) - 2,2 - dimethylcyclopropanecarboxamide having an optical purity of 99%.

m.p. 135°—136°C

$[\alpha]_{546}^{20}$ +99.5° (c=2, ethanol).

**Claims**

1. A method for the optical purification of an optically active 2,2 - dimethylcyclopropanecarboxamide by dissolving an optically active mixture of 2,2 - dimethylcyclopropanecarboxamide in which either one of the optically active isomers is present in excess, in a purification solvent with heating, cooling the solution to deposit the racemate, filtering off the racemate and then recovering said optically active isomer from the filtrate, which method is characterised in that the amount of the purification solvent present at the filtration stage is in the range of from 0.75 to 1.3 times inclusive as much as that required to dissolve the said excess of the optically active isomer at the filtration temperature, so as to form, at the filtration temperature, an essentially saturated solution containing the excess optically active isomer and to form crystals of the racemate which can be filtered off.

2. A method according to Claim 1, which includes the preliminary step of reacting 2,2 - dimethylcyclopropanecarboxylic acid in which either one of the optically active isomers is present in excess, with thionyl chloride, and then reacting the resulting product with ammonia to provide the said optically active mixture of 2,2 - dimethylcyclopropanecarboxamide in which either one of the optically active isomers is present in excess.

3. A method according to Claim 2, wherein the molar ratio of 2,2 - dimethylcyclopropanecarboxylic acid: thionyl chloride is from 1:1.02 to 1:1.3 inclusive.

4. A method according to Claim 2 or 3, wherein said ammonia is aqueous ammonia.

5. A method according to Claim 3 or 4, wherein said aqueous ammonia is a mixed solution with an organic, reaction solvent sparingly soluble in water.

6. A method according to Claim 5, wherein the said organic, reaction solvent is a ketone, halogenated hydrocarbon, or ester.

7. A method according to Claim 6, wherein the said organic, reaction solvent is selected from methyl ethyl ketone, methyl isobutyl ketone, cyclopentanone, cyclohexanone, dichloromethane, chloroform, dichloroethane, tetrachloroethane, dichlorobenzene, ethyl acetate, isopropyl acetate, methyl propionate and pentyl acetate.

8. A method according to any one of Claims 2 to 7, wherein the reaction with ammonia is carried out at a temperature in the range of from −10° to 15°C inclusive.

9. A method according to any preceding claim, wherein said purification solvent is selected from alcohols, ketones, esters, ethers, halogenated hydrocarbons and aqueous mixtures containing any of these solvents.

10. A method according to Claim 9, wherein the said purification solvent is selected from methanol, ethanol, isopropyl alcohol, butanol, acetone, methyl ethyl ketone, cyclopentanone, cyclohexanone, ethyl acetate, isopropyl acetate, methyl propionate, pentyl acetate, tetrahydrofuran, dioxane, dichloromethane, chloroform, dichlorethane, tetrachloroethane, dichlorobenzene and aqueous mixtures containing any of these solvents.

## Patentansprüche

1. Verfahren zur optischen Reinigung eines optisch aktiven 2,2 - Dimethylcyclopropancarboxamids durch Auflösen eines optisch aktiven Gemischs von 2,2 - Dimethylcyclopropancarboxamid, in welchem eines der optisch aktiven Isomeren im Überschuß vorliegt, in einem Reinigungslösungsmittel unter Erwärmen, Kühlen der Lösung zur Ausscheidung des Racemats, Abfiltrieren des Racemats und anschließend Reindarstellung des optisch aktiven Isomeren aus dem Filtrat, dadurch gekennzeichnet, daß die Menge an in der Filtrationsstufe vorhandenem Reinigungslösungsmittel das 0,75- bis einschließlich 1,3-fache der zum Auflösen des Überschusses an dem optisch aktiven Isomeren bei der Filtrationstemperatur erforderlichen Menge beträgt, um bei der Filtrationstemperatur eine im wesentlichen gesättigte Lösung mit dem überschüssigen optisch aktiven Isomeren und abfiltrierbare Kristalle des Racemats zu bilden.

2. Verfahren nach Anspruch 1, dadurch gekennzeichnet, daß in einer Vorstufe 2,2 - Dimethylcyclopropancarbonsäure, in welcher eines der optisch aktiven Isomeren im Überschuß vorhanden ist, mit Thionylchlorid und anschließend das erhaltene Produkt mit Ammoniak umgesetzt werden, um das optisch aktive Gemisch von 2,2 - Dimethylcyclopropancarboxamid mit einem der optisch aktiven Isomeren im Überschuß bereitzustellen.

3. Verfahren nach Anspruch 2, dadurch gekennzeichnet, daß das Molverhältnis 2,2 - Dimethylcyclopropancarbonsäure/Thionylchlorid 1:1,02 bis 1 zu einschließlich 1,3 beträgt.

4. Verfahren nach Anspruch 2 oder 3, dadurch gekennzeichnet, daß Ammoniak in Form wäßrigen Ammoniaks verwendet wird.

5. Verfahren nach Anspruch 3 oder 4, dadurch gekennzeichnet, daß das wäßrige Ammoniak aus einem Lösungsgemisch mit einem organischen, kaum wasserlöslichen Reaktionslösungsmittel besteht.

6. Verfahren nach Anspruch 5, dadurch gekennzeichnet, daß das organische Reaktionslösungsmittel aus einem Keton, halogenierten Kohlenwasserstoff oder Ester besteht.

7. Verfahren nach Anspruch 6, dadurch gekennzeichnet, daß das organische Reaktionslösungsmittel aus Methylethylketon, Methylisobutylketon, Cyclopentanon, Cyclohexanon, Dichlormethan, Chloroform, Dichlorethan, Tetrachlorethan, Dichlorbenzol, Ethylacetat, Isopropylacetat, Methylpropionat und Pentylacetat ausgewählt ist.

8. Verfahren nach einem der Ansprüche 2 bis 7, dadurch gekennzeichnet, daß die Reaktion mit Ammoniak bei einer Temperatur im Bereich von −10°C bis einschließlich 15°C durchgeführt wird.

9. Verfahren nach einem der vorhergehenden Ansprüche, dadurch gekennzeichnet, daß das Reinigungslösungsmittel aus Alkoholen, Ketonen, Estern, Ethern, halogenierten Kohlenwasserstoffen und wäßrigen Gemischen mit einem dieser Lösungsmittel ausgewählt ist.

10. Verfahren nach Anspruch 9, dadurch gekennzeichnet, daß das Reinigungslösungsmittel aus Methanol, Ethanol, Isopropanol, Butanol, Aceton, Methylethylketon, Cyclopentanon, Cyclohexanon, Ethylacetat, Isopropylacetat, Methylpropionat, Pentylacetat, Tetrahydrofuran, Dioxan, Dichlormethan, Chloroform, Dichlorethan, Tetrachlorethan, Dichlorbenzol und wäßrigen Gemischen mit einem dieser Lösungsmittel ausgewählt ist.

## Revendications

1. Procédé de purification optique d'un 2,2 - diméthylcyclopropanecarboxamide optiquement actif par dissolution, par chauffage, dans un solvant de purification d'un mélange optiquement actif de 2,2 - diméthylcyclopropanecarboxamide dans lequel l'un ou l'autre des isomères optiquement actifs est présent en excès, refroidissement de la solution pour faire déposer le racémate, filtration du racémate puis récupération dans le filtrat de l'isomère optiquement actif, procédé caractérisé en ce que la quantité de solvant de purification présent lors de l'étape de filtration est de 0,75 à 1,3 fois compris celle nécessaire pour dissoudre ledit excès d'isomère optiquement actif à la température de filtration, de façon à former, à la température de filtration, une solution pratiquement saturée contenant l'isomère optiquement actif en excès et à former des cristaux de racémate qui peuvent en être filtrés.

2. Procédé selon la revendication 1, qui comprend l'étape préliminaire de réaction d'un acide 2,2 - diméthylcyclopropanecarboxylique, dans lequel l'un ou l'autre des isomères optiquement actifs est présent en excès, avec du chlorure de thionyle, puis de réaction du produit résultant avec de l'ammoniac pour former ledit mélange optiquement actif de 2,2 - diméthylcyclopropanecarboxamide dans lequel l'un ou l'autre des isomères optiquement actifs est présent en excès.

3. Procédé selon la revendication 2, dans lequel le rapport molaire acide 2,2 - diméthylcyclopropane-carboxylique: chlorure de thionyl est de 1:1,02 à 1:1,3 compris.

4. Procédé selon la revendication 2 ou 3, dans lequel l'ammoniac est de l'ammoniaque.

5. Procédé selon la revendication 3 ou 4, dans lequel ladite ammoniaque est une solution mixte avec un solvant réactionnel organique faiblement soluble dans l'eau.

6. Procédé selon la revendication 5, dans lequel ledit solvant réactionnel organique est une cétone, un hydrocarbure halogéné ou un ester.

7. Procédé selon la revendication 6, dans lequel ledit solvant réactionnel organique est choisi entre la méthyléthylcétone, la méthylisobutylcétone, la cyclopentanone, la cyclohexanone, le dichlorométhane, le chloroforme, le dichloroéthane, le tétrachloroéthane, le dichlorobenzène, l'acétate d'éthyle, l'acétate d'isopropyle, le propionate de méthyle ou l'acétate de pentyle.

8. Procédé selon l'une quelconque des revendications 2 à 7, dans lequel la réaction avec l'ammoniac est effectuée à une température comprise entre −10° et +15°C compris.

9. Procédé selon l'une quelconque des revendications précédentes, dans lequel ledit solvant de purification est choisi entre les alcools, les cétones, les esters, les éthers, les hydrocarbures halogénés et les mélanges aqueux contenant l'un quelconque de ces solvants.

10. Procédé selon la revendication 9, dans lequel ledit solvant de purification est choisi entre le méthanol, l'éthanol, l'alcool isopropylique, le butanol, l'acétone, la méthyléthylcétone, la cyclopentanone, la cyclohexanone, l'acétate d'éthyle, l'acétate d'isopropyle, le propionate de méthyle, l'acétate de pentyle, le tétrahydrofuranne, le dioxanne, le dichlorométhane, le chloroforme, le dichloroéthane, le tétrachloroéthane, le dichlorobenzène et les mélanges aqueux contenant l'un quelconque de ces solvants.